# EUROPEAN PATENT APPLICATION

(11) **EP 1 829 855 A1**
(43) Date of publication of application: **05.09.2007**
(21) Application number: 05806000.5
(22) Date of filing: 02.11.2005
(51) Int. Cl.: C07C 211/57

(54) **NOVEL AROMATIC TERTIARY AMINES AND UTILIZATION THEREOF AS ORGANIC ELECTRONIC FUNCTIONAL MATERIAL**

(30) Priority: 08.11.2004 JP 2004324334; 16.03.2005 JP 2005074464
(71) Applicant: Bando Chemical Industries, Ltd., Kobe-shi, Hyogo 652-0883 (JP)
(72) Inventor: NAKAYAMA, Masami, Bando Chemical Industries, Ltd., Kobe-shi Hyogo 652-0883 (JP); TSUBAKI, Tomoyuki, Bando Chemical Industries, Ltd., Kobe-shi Hyogo 652-0883 (JP)
(74) Representative: Cresswell, Thomas Anthony
(86) International application number: PCT/JP2005/020498
(87) International publication number: WO 2006/049316

(57) **Abstract**

The invention provides an aromatic tertiary amine represented by the general formula (I) 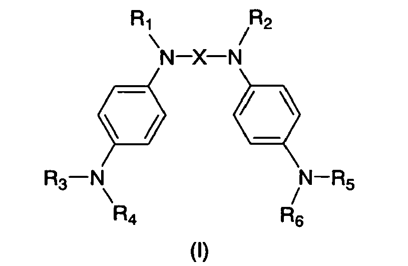 wherein X represents an aromatic divalent group which is 1,4-phenylene, a 1,4-naphthalenediyl or a 9,10-anthracenediyl group, and which may have substituents thereon; and R₁ to R₆ are each independently represent an aromatic monovalent group which may have substituents thereon, preferably a phenyl or a naphthyl group. The aromatic tertiary amine is useful as an organic electronic functional material, and can be suitably used as a hole injecting agent in a variety of electronic devices such as an electroluminescence element.

## Description

### Field of the Invention

This invention relates to novel aromatic tertiary amines useful as amorphous electronic materials and their use as organic electronic functional materials. More particularly, the invention relates to novel aromatic tertiary amines which remain amorphous at normal temperatures or higher so that they can form thin films by themselves, and in addition, which are highly heat resistant so that they are suitable for use as an organic electronic functional material, in particular, as a hole injecting agent in a variety of electronic devices such as organic electroluminescence elements, organic photosensitive elements, organic solar battery elements or field-effect transistors. The invention further relates to use of the aromatic tertiary amines as an organic electronic functional material, in particular, as a hole injecting agent in an organic electroluminescence element.

### Background Art

Among organic materials having a photoelectric function which produce electroconductivity or electric charges when being irradiated, that is, organic electronic functional materials, most of the low molecular weight organic compounds are incapable of forming thin film by themselves. Accordingly, when thin film is to be formed with such known low molecular weight organic compounds, they are dispersed in a binder resin (that is, diluted with a binder resin), and the resulting dispersion is applied onto a substrate to form thin film. Thus, the conventional organic electronic functional materials comprised of low molecular weight organic compounds are influenced by the binder resin which forms a matrix, but also they are diluted with the binder resin so that they cannot exhibit sufficiently the properties that they originally possess.

In addition, if the known low molecular weight organic compounds having a photoelectric function form thin film that is relatively stable at normal temperatures with the aid of a binder resin, they have low glass transition temperatures so that the film is poor in heat resistance and is not suitable for practical use. Accordingly, the development of electronic materials that are amorphous at normal temperatures and are capable of forming film by themselves has been pushed on with in recent years.

On the other hand, as described in JP-A-Nos. 6-1972 and 7-90256, among a variety of electronic devices, an organic electroluminescence element in particular can be driven by a low voltage with high efficiency to emit light at a high luminance, as well as it can be made thin. Accordingly, in recent years, the investigation to put the organic electroluminescence element to practical use as display devices as well as backlights or illumination devices is pushed forward.

The electroluminescence element is comprised usually of a transparent substrate such as a glass substrate having an anode made of a transparent electrode such as an ITO membrane (indium oxide-tin oxide membrane) laminated thereon, and a hole transporting layer, an emitting layer and a cathode made of a metal electrode laminated on the anode in this order. The anode and the cathode are connected with an external power source. In some cases, the hole transporting layer is divided into a hole injecting layer and a hole transporting layer, and laminated as such, and in some cases, an electron transporting layer is laminated between the emitting layer and the cathode. Many other layer structures to form organic electroluminescence elements are known, as described in, for example, JP-A No. 6-1972.

In such an organic electroluminescence element, the hole transporting layer adheres to the anode, and transports holes from the anode to the emitting layer while blocking electrons, whereas the electron transporting layer adheres to a cathode, and transports electrons from the cathode to the emitting layer. Thus, when an electron injected from the cathode and a hole injected from the anode recombine in the emitting layer, light is emitted and radiated outside through the transparent electrode (anode) and the transparent substrate.

Such aromatic tertiary amines as 4,4'-bis(N-(3-methylphenyl)-N-phenylamino)biphenyl (TPD) (as described, for example, in JP-A-No. 7-90256) and 4,4'-bis(N-(1-naphthyl)-N-phenylamino))biphenyl (α-NPD) (as described, for example, in JP-A- No. 5-234682) are known as organic electronic functional materials used in the hole transporting layer, or hole transporting agents, for the conventional organic electroluminescence elements. However, the organic electroluminescence element provided with the hole transporting layer making use of these aromatic tertiary amines as a hole transporting agent needs high voltage to work.

Under these circumstances, for example, 4,4',4"-tris(N,N-phenyl-m-tolylamino)triphenylamine (m-MTDATA) represented by the formula (1) 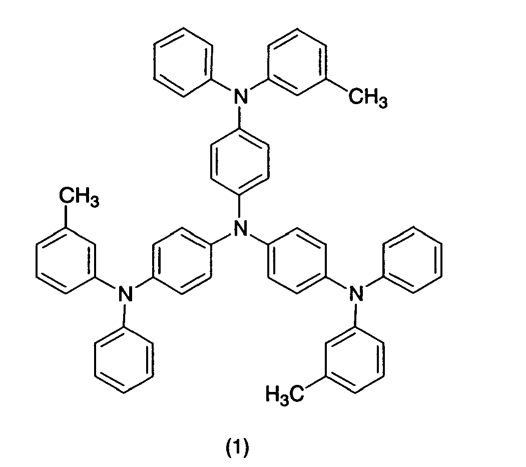 (as described in JP-A-No. 1-224353) and 4,4',4"-tris(N,N-(2-naphthyl)-phenylamino)triphenylamine (2-TNATA) represented by the formula (2) (as described in JP-A-No. 8-291115) 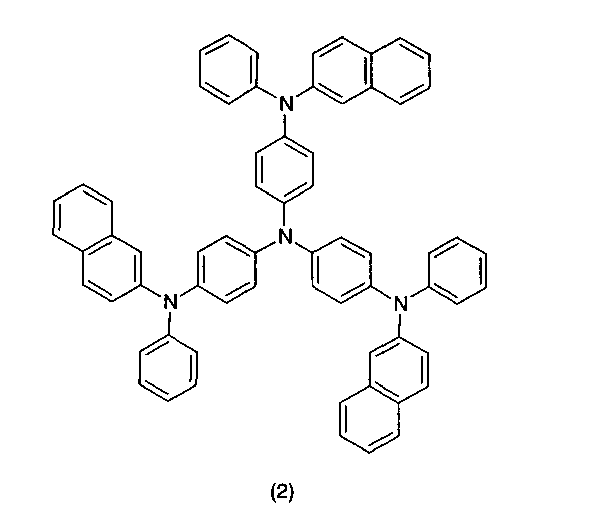 have been proposed. Further, 4,4',4"-tris(N,N-(1-naphthyl)phenylamino)-triphenylamine (1-TNATA) has been proposed as a hole transporting agent used in an organic electroluminescence element (as described in JP-A-No. 5-234681 and "Monthly Display", October, Separate Volume, pp. 28-35, 1998).

However, these triphenylamines, for example, 4,4',4"-tris(N,N-phenyl-m-tolylamino)triphenylamine (m-MTDATA) has a glass transition temperature of about 77°C so that it is difficult to use the compound in practical electronic devices, and on the other hand, 4,4',4"-tris-(N,N-(2- or 1-naphthyl)phenylamino)triphenylamine (2- or 1-TNATA) has a glass transition temperature of about 110°C and forms a heat resistant amorphous film, but it is apt to form crystals and it is hard to say that it remains amorphous stably.

Therefore, it is strongly demanded to develop an organic electronic functional material which is capable of forming thin film by itself and excellent in heat resistance, and in addition, which can be used as a hole injecting agent in combination with a hole transporting agent to provide an organic electroluminescence element which can be driven at a low voltage.

The invention has been achieved to solve the problems involved in the conventional organic materials as amorphous electronic materials. Thus, it is an object of the invention to provide a novel aromatic tertiary amine which can form stable amorphous film at ordinary temperature or higher by itself without the aid of binder resins, and in addition, which has a high glass transition temperature, and hence has excellent heat resistance, and accordingly which can be suitably used as a hole injecting agent which makes it possible to transport holes from an anode to a hole transporting layer, and injects the holes into an emission layer, at a low voltage.

It is a further object of the invention to provide an organic electronic functional material comprising such an aromatic tertiary amine, a hole injecting agent comprising such an organic electronic functional material, and further an organic electroluminescence element provided with a hole injecting layer comprising such a hole transporting agent.

### Disclosure of the Invention

The invention provides an aromatic tertiary amine represented by the general formula (I) 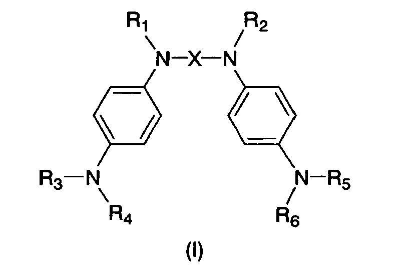 wherein X is an aromatic divalent group represented by the general formula (II), (III) or (IV) 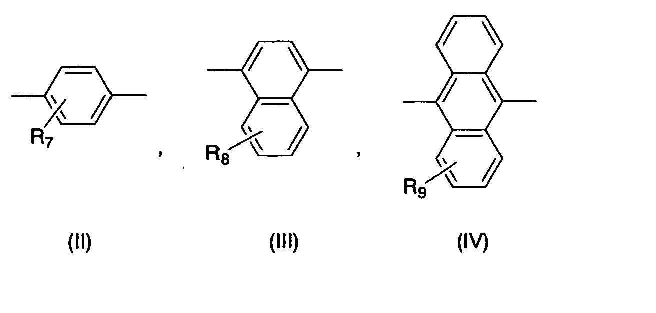 wherein R₇ to R₉ each independently represent a hydrogen atom, an alkyl group of 1 to 6 carbons or an alkoxyl group of 1-4 carbons, and R₁ to R₆ each independently represent a monovalent group (V), (VI), (VII), (VIII), (IX) or (X) 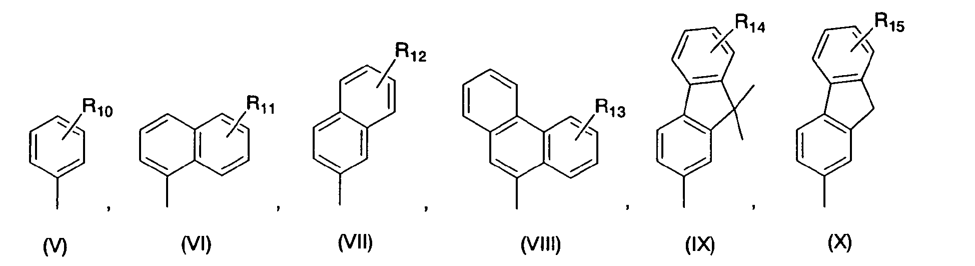 wherein R₁₀ to R₁₅ each independently represent a hydrogen atom, an alkyl group of 1 to 6 carbons or an alkoxyl group of 1 to 4 carbon atoms, provided that all of R₁ to R₆ are each a phenyl group, X is not a 1,4-phenylene group.

According to the invention, the first one of the particularly preferred aromatic tertiary amines is such that it is represented by the general formula (I) in which R₁, R₂, R₃ and R₅ each independently represent a 1- or 2-naphthyl group, and the second one of the particularly preferred aromatic tertiary amines is such that it is represented by the general formula (I) in which all of R₁, R₂, R₃, R₄, R₅ and R₆ are each a phenyl or a tolyl group. In these first and second ones of the particularly preferred aromatic tertiary amines of the invention, the aromatic divalent group X is a 1,4-phenylene, a 1,4-naphthalenediyl or a 9,10-anthracenediyl group. However, when all of R₁ to R₆ are each a phenyl group, the group X is not a 1,4-phenylene group.

The invention further provides an organic electronic functional material comprising the aromatic tertiary amine, a hole injecting agent comprising such an organic electronic functional material, and an organic electroluminescence element provided with a hole injecting layer comprising such a hole injecting agent.

### Brief Description of the Drawings

Fig.1 is a sectional view of an example of an organic electroluminescence element of the invention;
Fig. 2 is a DSC chart of 1,4-bis[N-(4-(N-2-naphthyl-N-phenylamino)-phenyl)-N-2-naphthylamino]benzene of the invention;
Fig. 3 is a TG/DTA chart of 1,4-bis[N-(4-(N-2-naphthyl-N-phenylamino)phenyl)-N-2-naphthylamino]benzene of the invention;
Fig. 4 is a CV chart of 1,4-bis[N-(4-(N-2-naphthyl-N-phenylamino)-phenyl)-N-2-naphthylamino]benzene of the invention;
Fig. 5 is a graph showing the voltage-luminance characteristics of an organic electroluminescence element of the invention compared with the voltage-luminance characteristics of an organic electroluminescence element of a comparative example;
Fig. 6 is a graph showing the voltage-current density characteristics of an organic electroluminescence of the invention compared with the voltage-current density characteristics of an organic electroluminescence of a comparative example;
Fig. 7 is an FT-IR spectrum of 1,4-bis[N-(4-diphenylaminophenyl)-N-phenylamino] naphthalene;
Fig. 8 is a DSC chart of 1,4-bis[N-(4-diphenylaminophenyl)-N-phenylamino] naphthalene;
Fig. 9 is a TG/DTA chart of 1,4-bis[N-(4-diphenylaminophenyl)-N-phenylamino]naphthalene; and
Fig. 10 is a CV chart of 1,4-bis[N-(4-diphenylaminophenyl)-N-phenylamino] naphthalene.

### Best Mode for Carrying out the Invention

The aromatic tertiary amine of the invention is represented by the general formula (I) 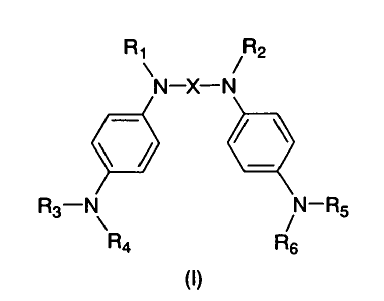 wherein X is an aromatic divalent group represented by the general formula (II), (III) or (IV) 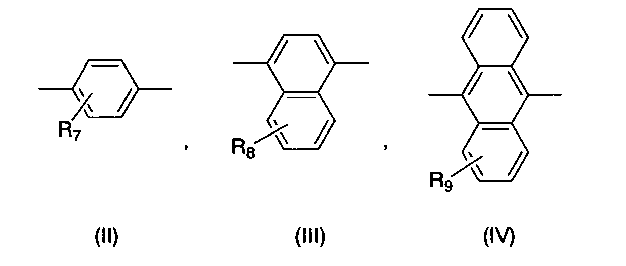 wherein R₇ to R₉ each independently represent a hydrogen atom, an alkyl group of 1 to 6 carbons or an alkoxyl group of 1-4 carbons, and R₁ to R₆ each independently represent a monovalent group (V), (VI), (VII), (VIII), (IX) or (X) 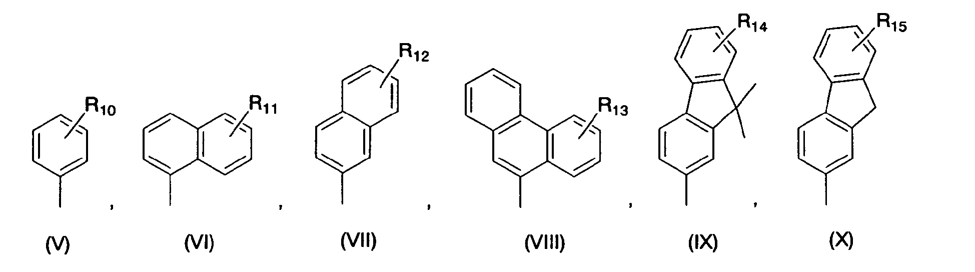 wherein R₁₀ to R₁₅ each independently represent a hydrogen atom, an alkyl group of 1 to 6 carbons or an alkoxyl group of 1 to 4 carbon atoms, provided that all of R₁ to R₆ are each a phenyl group, X is not a 1,4-phenylene group.

When the groups R₇ to R₉ are each an alkyl group of 1 to 6 carbons, examples of such alkyl groups include, for example, a methyl, an ethyl, a propyl, a butyl and a hexyl group. When the alkyl group has three or more of carbons, it may be either linear or branched. When the groups R₇ to R₉ are each an alkoxyl group of 1 to 4 carbons, examples of such alkoxyl groups include, for example, a methoxyl, an ethoxyl, a propoxyl and a butoxyl group. When the alkyl group in the alkoxyl group has three or more of carbons, the alkyl group may be either linear or branched.

Similarly, when the groups R₁₀ to R₁₅ are each an alkyl group of 1 to 6 carbons, examples of such alkyl groups include, for example, a methyl, an ethyl, a propyl, a butyl and a hexyl group. When the alkyl group has three or more of carbons, it may be either linear or branched. When the groups R₁₀ to R₁₅ are each an alkoxyl group of 1 to 4 carbons, examples of such alkoxyl groups include, for example, a methoxyl, an ethoxyl, a propoxyl and a butoxyl group. When the alkyl group in the alkoxyl group has three or more of carbons, the alkyl group may be either linear or branched.

In the aromatic groups represented by the general formula (II) to (X), when the groups R₇ to R₁₅ are substituents other than a hydrogen atom, for example, the groups R₇ to R₁₅ are each an alkyl or an alkoxyl group, such substituents may be carried on any of the benzene rings which form the aromatic group, and the number of the substituents is within the range of 1 to 4. Further, when the group represented by the general formula (II) to (X) has two or more of substituents in this way, the substituents may independently be any one of the above-mentioned. However, according to the invention, it is preferred that the groups R₇ to R₁₅ are each a hydrogen atom or an alkyl group, and when they are an alkyl group, it is preferably a methyl group.

In particular, the first one of the preferred aromatic tertiary amines of the invention is such that it is represented by the general formula (I) in which R₁, R₂, R₃ and R₅ are each independently a 1- or a 2-naphthyl group. That is to say, the first one of the preferred aromatic tertiary amines of the invention is represented by the general formula (Ia) 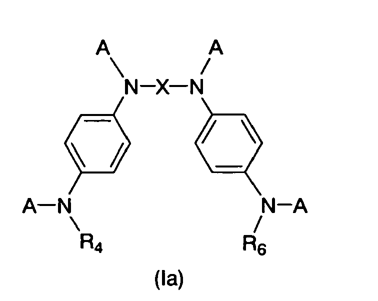 wherein A is independently a 1- or a 2-naphthyl group, and X, R₄ and R₆ are the same as the above.

Among these aromatic tertiary amines of the invention, the particularly preferred first one is such that it is represented by the general formula (Ia) in which R₄ and R₆ are each a phenyl, a tolyl (i.e., o-, m- or p-tolyl) or a naphthyl group (i.e., 1- or 2- naphthyl group), and preferably in which R₄ and R₆ are each a phenyl group, and the group X is a 1, 4-phenylene group, a 1,4-naphthalenediyl group or a 9,10-anthracenediyl group.

Therefore, examples of the first one of the preferred aromatic tertiary amines of the invention include:
1,4-bis[N-(4-(N-naphthyl-N-phenylamino)phenyl)-N-naphthylamino]-benzene,
1, 4-bis [N-(4-(N-naphthyl-N-tolylamino)phenyl)-N-naphthylamino] benzene,
1,4-bis[N-(4-(N,N-dinaphthylamino)phenyl)-N-naphthylamino]benzene,
1,4-bis[N-(4-(N-naphthyl-N-phenylamino)phenyl)-N-naphthylamino]-naphthalene,
1,4-bis[N-(4-(N-naphthyl-N-tolylamino)phenyl)-N-naphthylamino]-naphthalene,
1,4-bis [N-(4-(N,N-dinaphthylamino)phenyl)-N-naphthylamino]-naphthalene,
9,10-bis[N-(4-(N-naphthyl-N-phenylamino)phenyl)-N-naphthylamino]-nanthracene,
9,10-bis[N-(4-(N-naphthyl-N-tolylamino)phenyl)-N-naphthylamino]-anthracene, and
9,10-bis[N-(4-(N,N-dinaphthylamino)phenyl)-N-naphthylamino]-anthracene, although the first one of the preferred aromatic tertiary amines are not limited to those enumerated above.

The most preferred one of the first ones of the preferred aromatic tertiary amines of the invention is 1,4-bis[N-4-(N-2-naphthyl-N-phenylamino)phenyl-N-2-naphthylamino]benzene represented by the formula (3) below

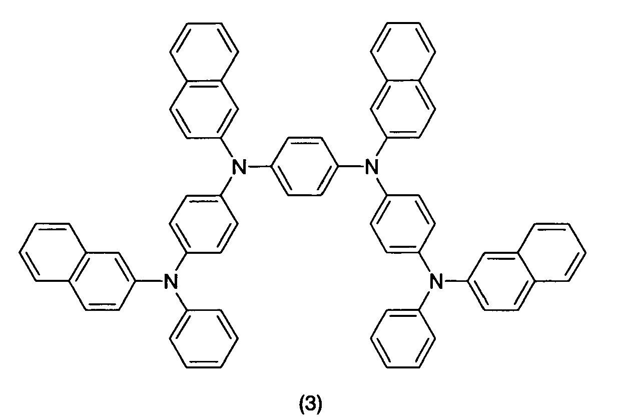

The first one of the preferred aromatic tertiary amines of the invention, for example, the aromatic tertiary amine represented by the above-mentioned formula (3), is obtained as shown in the scheme below. N,N'-di-2-naphthyl-1,4-phenylenediamine (4) is reacted with iodobenzene (5) in an inert solvent such as mesitylene in the presence of a base such as potassium carbonate and copper powder to obtain N,N'-di-2-naphthyl-N-phenyl-1,4-phenylenediamine (6). Then, N,N'-di-2- naphthyl-N-phenyl-1,4-phenylenediamine (6) is reacted with 1,4-dibromobenzene (7) in an inert solvent in the presence of a strong base like sodium t-butoxide and a catalyst such as palladium acetate and tri-t-butylphosphine, thereby providing the aromatic tertiary amine represented by the formula (3).

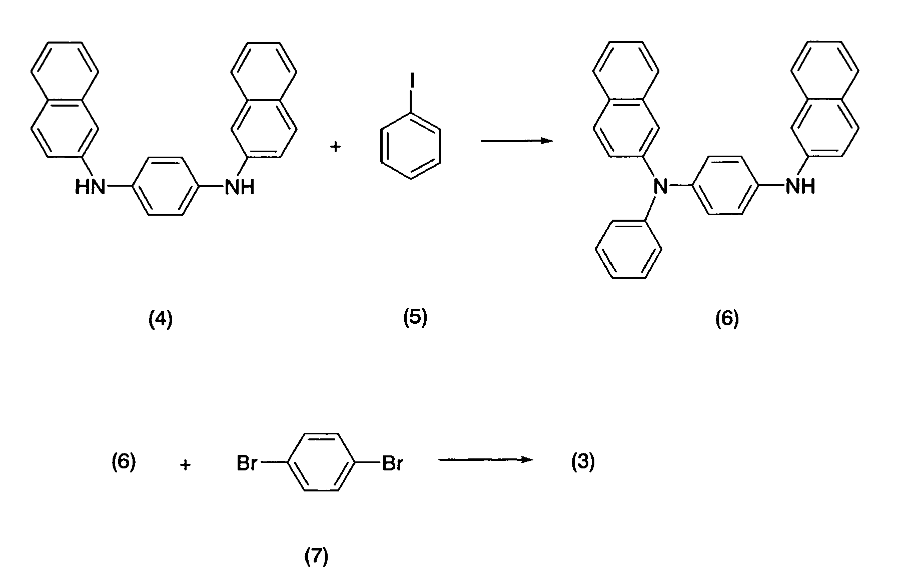

The second one of the preferred aromatic tertiary amines of the invention is such that it is represented by the general formula (I) in which R₁, R₂, R₃, R₄, R₅ and R₆ each independently represent a phenyl or a tolyl group (i.e., o-, m- or p-tolyl group), and more preferably, in which R₁, R₂, R₃, R₄, R₅ and R₆ each independently represent a phenyl or a tolyl group and the aromatic divalent group X is a 1,4-phenylene, a 1,4-naphthalenediyl or a 9,10-anthracenediyl group, provided that all of the groups R₁, R₂, R₃, R₄, R₅ and R₆ are each a phenyl group, the group X is not a 1,4-phenylene group.

Accordingly, examples of such second ones of the preferred aromatic tertiary amines of the invention include, for example,
1, 4-bis [N-(4-ditolylaminophenyl)-N-tolylamino] benzene,
1,4-bis[N-(4-phenyltolylaminophenyl)-N-tolylamino]benzene,
1,4-bis[N-(4-diphenylaminophenyl)-N-phenylamino]naphthalene,
1, 4-bis [N-(4-ditolylaminophenyl)-N-tolylamino] naphthalene,
1, 4-bis [N-(4-phenyltolylaminophenyl)-N-tolylphenylamino] naphthalene,
1,4- bis[N-(4-phenyltolylaminophenyl)-N-phenylamino]naphthalene, and
9,10- bis[N-(4-diphenylaminophenyl)-N-phenylamino]anthracene.

In particular, the second one of the preferred aromatic tertiary amines of the invention is such that it is represented by the general formula (I) in which all of the groups R₁, R₂, R₃, R₄, R₅ and R₆ are each a phenyl group, which is represented by the general formula (Ib) 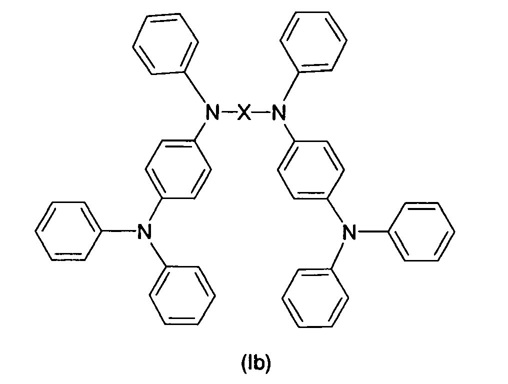 wherein the aromatic divalent group X is a 1,4-naphthalenediyl or a 9,10-anthracenediyl group

The most preferred one of the second ones of the preferred aromatic tertiary amines of the invention is 1,4-bis[N-(4-diphenylaminophenyl)-N-phenylamino]naphthalene represented by the formula (8)

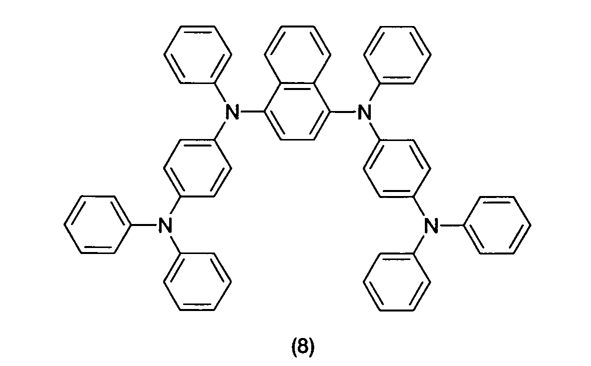

These aromatic tertiary amines are obtained in the same manner as 1,4-bis[N-4-(N-2-naphthyl-N-phenylamino)phenyl-N-2-naphthylamino]-benzene mentioned hereinbefore. Taking the aromatic tertiary amine represented by the formula (8) as an example, it is obtained as shown in the scheme below. N,N'-diphenyl-1,4-phenylenediamine (9) is reacted with iodobenzene(5) in an inert solvent such as mesitylene in the presence of a base such as potassium carbonate and copper powder to obtain N,N',N'-triphenyl-1,4-phenylenediamine (10), which is then reacted with 1,4-dibromonaphthalene (11) in an inert solvent in the presence of a strong base like sodium t-butoxide and a catalyst such as palladium t-butylphosphinebromide dimer, thereby providing the aromatic tertiary amine represented by the formula (8).

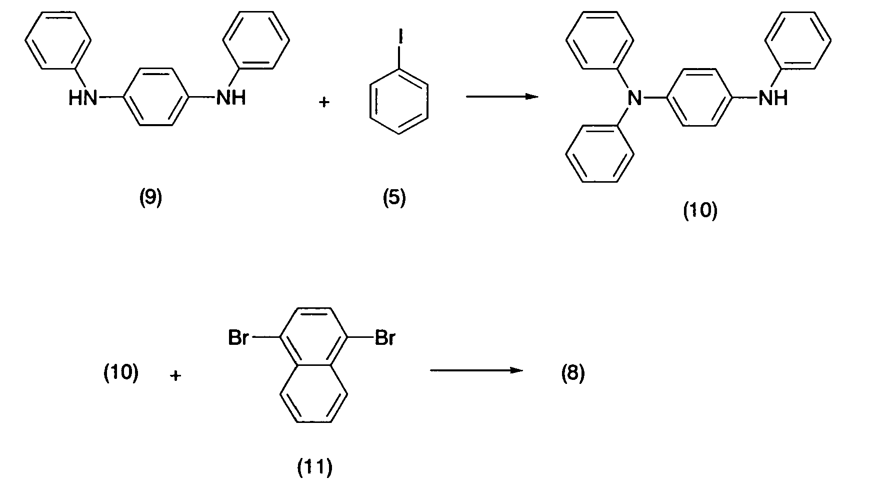

Since the aromatic tertiary amine of the invention shows no clear peaks in powder X-ray diffraction measurement, it has no anisotropy and is amorphous, and accordingly, it is capable of forming amorphous film by itself at normal temperatures or higher without the aid of a binder resin, but also it has a high glass transition temperature, and hence it is highly heat resistant. Accordingly, for example, an organic electroluminescence element that can be driven at a low voltage and has an outstanding durability is obtained by placing a hole injecting layer making use of such an organic electronic functional material as a hole injecting agent between an anode and a hole transporting layer. However, the organic electronic functional material of the invention may be formed to thin film using a binder resin.

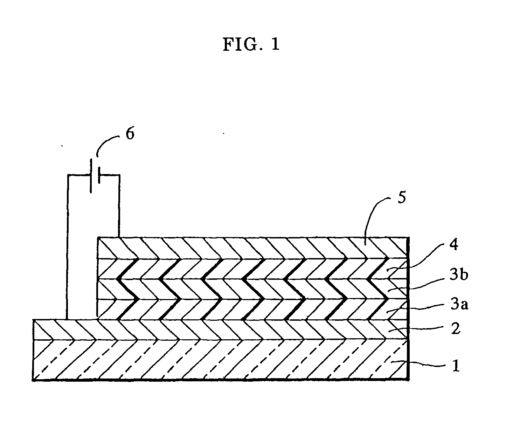

As a preferred example is shown in Fig. 1, the electroluminescence element of the invention is comprised of a transparent substrate 1 made of glass, for example, having an anode 2 made of a transparent electrode such as an ITO membrane (indium oxide-tin oxide membrane) laminated thereon, and a hole injecting layer 3a, a hole transporting layer 3b, an emitting layer 4 and a cathode 5 made of a metal or a compound thereof laminated on the anode in this order. The anode and the cathode are connected with an external power source 6. Thus, holes are readily injected to an emission layer through the hole injecting layer and the hole transporting layer so that the electroluminescence element can be driven at a low voltage. Electrons are injected from the cathode. Thus, the electrons injected from the cathode and the holes injected from the anode recombine in the emitting layer, thereby light is emitted and radiated outside through the transparent electrode (anode) and the transparent substrate.

In the invention, in some cases, an electron transporting layer may be laminated between the emitting layer and the cathode. Further, a blocking layer may be provided in order to prevent extra electrons from passing through the cathode. As mentioned above, the electroluminescence element of the invention is not specifically limited in layer structures.

The electroluminescence element of the invention is featured in that it has a hole injecting layer formed of the organic electronic functional material comprising the aromatic tertiary amine of the invention. The organic electronic functional material of the invention is capable of forming amorphous film by itself, and accordingly, a hole injecting layer can be formed by vacuum evaporating the organic electronic functional material onto the transparent electrode using a suitable vacuum evaporation device. The thickness of hole injecting layer is usually in the range of 10 nm to 200 nm, preferably in the range of 20 nm to 80 nm.

However, the organic electronic functional material may be dissolved in a suitable organic solvent, and if needed, together with an appropriate binder resin, to prepare a coating composition, and the coating composition may be applied onto the anode with an appropriate coating means such as a spin coat method, and then dried, thereby preparing a hole injecting layer. Also in this case, the thickness of the hole injecting layer is the same as mentioned above.

Then, a hole transporting layer is formed of a hole transporting agent such as α-NPD by a conventional method on the hole injecting layer thus formed, and then an emitting layer and a cathode is laminated on the hole transporting layer, thereby providing an organic electroluminescence element.

In the organic electroluminescence element of the invention, the layers except the hole injecting layer mentioned above, that is, a transparent substrate, a hole transporting layer, an anode, an emitting layer, an electron transporting layer and a cathode, may be made of any conventionally known materials. For example, an anode or a transparent electrode may be made of indium oxide-tin oxide (ITO), and a cathode may be made of a metal such as aluminum, magnesium, indium or silver, or an alloy of these metals, such as Al-Mg alloy, Ag-Mg alloy, or lithium fluoride. A transparent substrate is usually made of glass.

As the hole transporting agent, any known low molecular weight organic compounds, such as α-NPD and TPD mentioned hereinbefore, and 4,4',4"-tris(N-(3-methylphenyl)-N-phenylamino)triphenylamine (MTDATA) and the like are used. The thickness of the hole transporting layer is usually in the range of 10-200 nm.

Tris(8-quinolinol)aluminum (Alq₃), for example, is used for an emitting layer. The thickness of the emitting layer is usually in the range of 10-200 nm. When the organic electroluminescence element contains an electron transporting layer, the thickness thereof is usually in the range of 10-200 nm.

### Industrial Applicability

The novel aromatic tertiary amine of the invention represented by the general formula (I) has a better amorphous property at ordinary temperature or higher as well as a higher glass transition temperature, that is, higher heat resistance, than the conventionally known aromatic tertiary amines. Accordingly, the aromatic tertiary amine of the invention can be suitably used as an organic electronic functional material or a hole injecting agent which has excellent amorphous property and heat resistance in a variety of electronic devices, for example, in an organic electroluminescence element, an organic photoconductive element, an organic solar battery element, a field effect transistor, and the like. In particular, the use of the aromatic tertiary amine of the invention as a hole injecting agent provides an organic electroluminescence element that can be driven at a low voltage.

### Examples

The invention is described in more detail with reference to examples, however, the invention is not limited thereto.

### Example 1

### (Synthesis of N,N'-di-2-naphthyl-N-phenyl-1,4-phenylenediamine (6))

50.0 g of N,N'-di-2-naphthyl-1,4-phenylenediamine (4), 21.2 g of iodobenzene, 38.7 g of potassium carbonate and 12.0 g of copper powder and 100 mL of mesitylene as a reaction solvent were placed in a 500 mL capacity four necked glass flask, and the reaction was carried out at a refluxing temperature of 160°C to 165°C for 10 hours under a nitrogen atmosphere. After the reaction, the resultant reaction mixture was extracted with toluene and the toluene solution was concentrated. A mixed solvent of toluene/hexane was added to the resulting concentrate and the resulting mixture was subjected to silica gel chromatography to obtain fractions containing the reaction product. The fractions were concentrated to provide a viscose liquid, which was dried at a temperature of 120°C under reduced pressure to provide 20.1 g of the desired product in a yield of 66%.

### (Synthesis of 1,4-bis[N-(4-(N-2-naphthyl-N-phenylamino)phenyl)-N-2-naphthylamino]benzene (3))

20.0 g of N,N'-di-2-naphthyl-N-phenyl-1,4-phenylenediamine (6), 5.4 g of 1,4-dibromobenzene, 5.2 g of sodium t-butoxide, 0.005 g of palladium acetate and 0.02 g of tri-t-butylphosphine were dissolved in 100 mL of o-xylene. The reaction was carried out at a temperature of 120°C for 5 hours under a nitrogen atmosphere. After the reaction, the resulting reaction mixture was extracted with a mixture of water/toluene and the organic layer was dehydrated over anhydrous magnesium sulfide, followed by concentration, to provide yellow solid. The yellow solid was recrystallized from tetrahydrofuran, and was then subjected to sublimation purification, thereby providing 8.0 g of 1,4-bis[N-(4-(N-2-naphthyl-N-phenylamino)phenyl)-N-2-naphthylamino]benzene having a purity of 99.7%. The yield was 36%.

| Elemental analysis (%): | | | |
|---|---|---|---|
| | C | H | N |
| Calculated: | 88.76 | 5.32 | 5.92 |
| Measured: | 88.61 | 5.26 | 6.00 |

### Differential scanning calorimetry (DSC) :

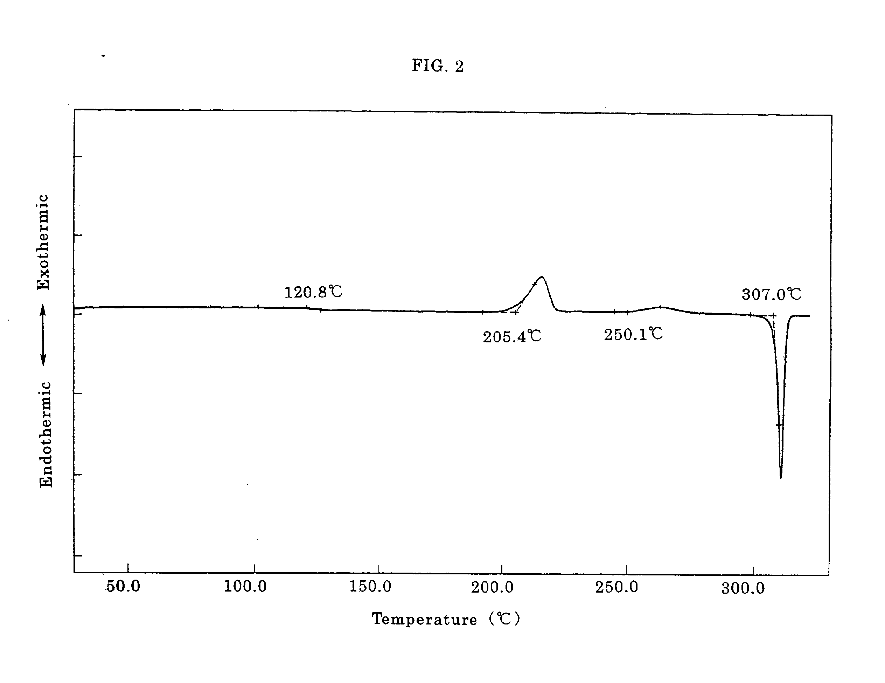

As the DSC chart is shown in Fig. 2, the compound was found to have a glass transition temperature (Tg) of 120.8°C, showing that the compound is excellent in heat resistance and capable of forming amorphous film at ordinary temperature or higher.

### Thermogravimetric measurement/Differential calorimetry (TG/DTA):

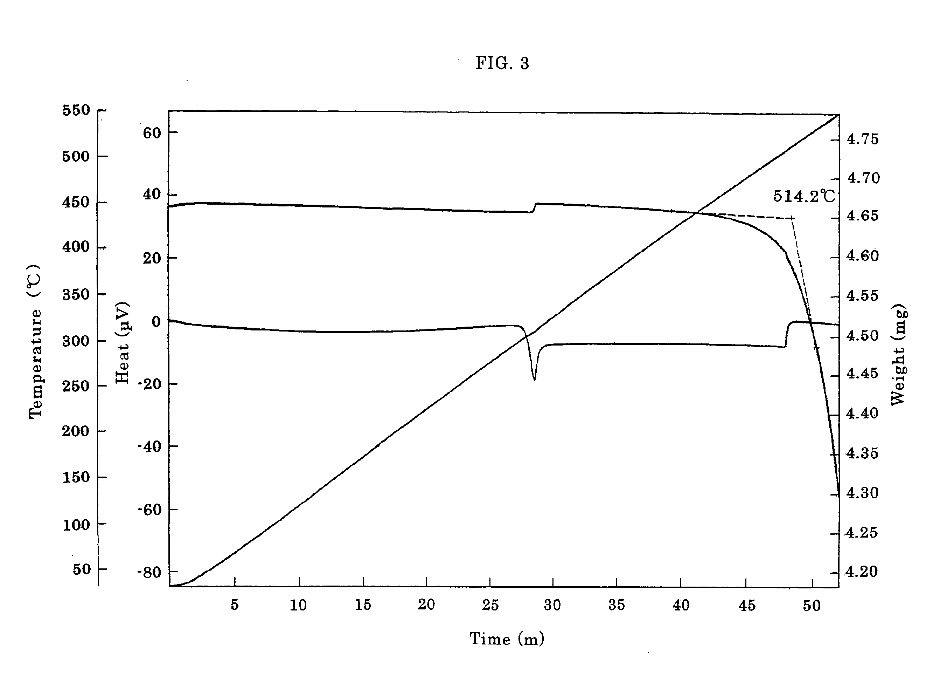

As the results of measurement is shown in Fig. 3, the compound was found to have a decomposition temperature of not less than 510°C, showing that the compound is excellent in heat stability.

### Cyclic voltammetry (CV):

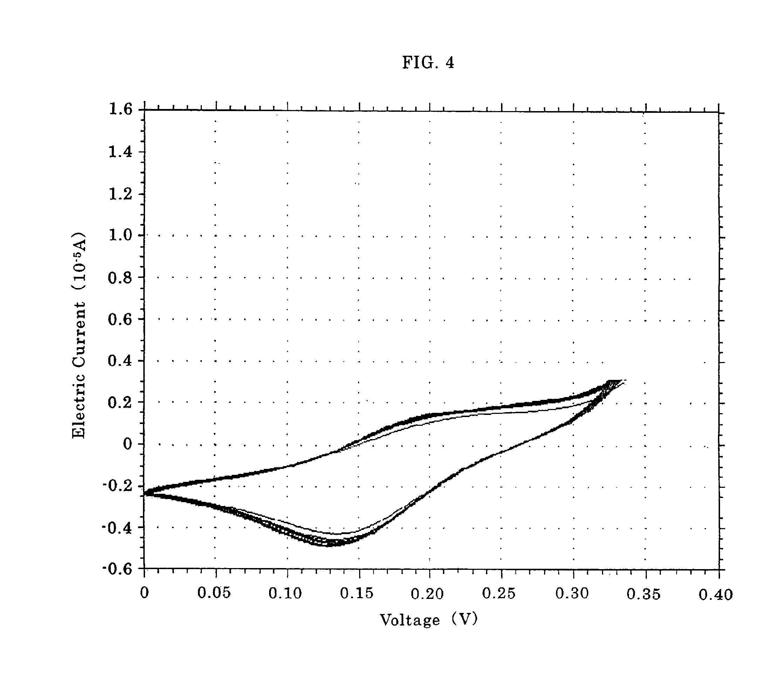

As the CV chart is shown in Fig. 4, the compound was found to have an oxidation potential of 0.16 V (vs. Ag/Ag⁺), and in addition, the compound was found to be excellent in repetition stability and reversibility in oxidation-reduction process.

### Example 2

A hole injecting layer (50nm thick) formed of 1,4-bis[N-(4-(N-2-naphthyl-N-phenylamino)phenyl)-N-naphthylamino]-benzene (3) was laminated on an ITO transparent electrode (anode). A hole transporting layer (10nm thick) was formed of 4,4'-bis(N-(1-naphthyl)-N-phenylamino)]biphenyl (α-NPD) on the hole injecting layer. An emission layer (75nm thick) was formed of tris(8-quinolinol) aluminum (Alq₃) on the hole transporting layer by a vacuum evaporation method. Further, lithium fluoride (0.5nm thick) and aluminum (100nm thick) were vacuum evaporated in this order as a cathode on the emission layer, thereby preparing an organic electroluminescence element.

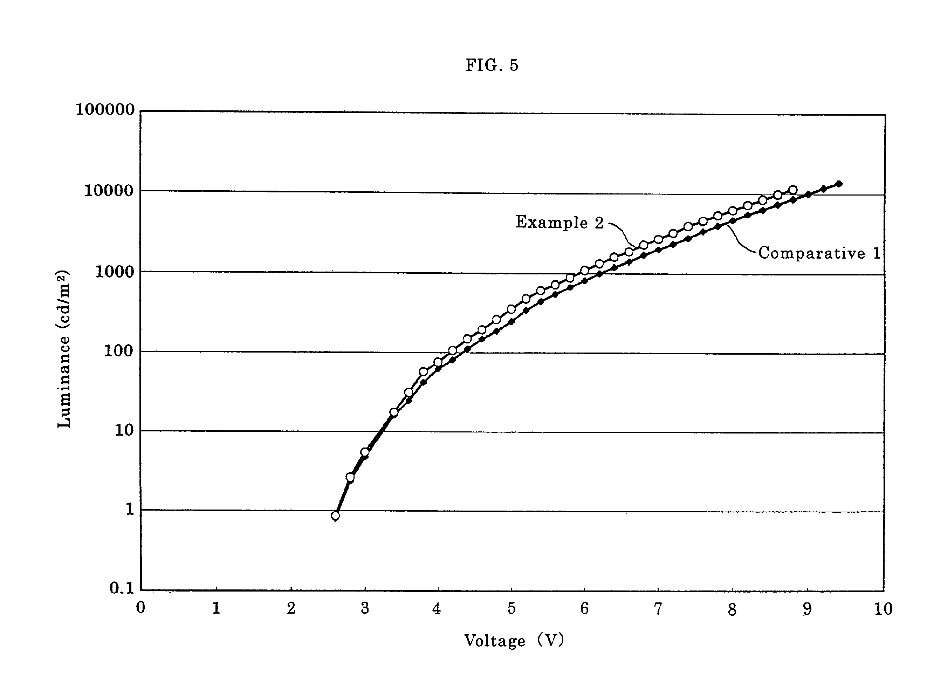

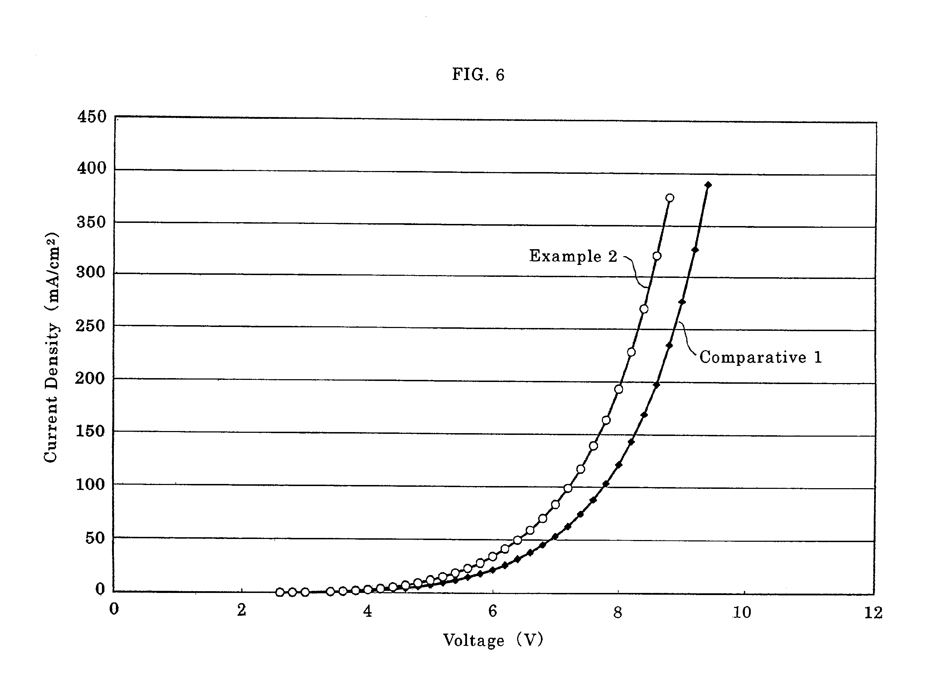

The characteristics of the organic electroluminescence element were examined at a fixed current and at a fixed luminance. The results are shown in Table 1. The voltage-luminance characteristics of the organic electroluminescence element are shown in Fig. 5, and the voltage-current density characteristics of the organic electroluminescence element are shown in Fig. 6. These results illustrate that the organic electroluminescence element of the invention can be driven at a low voltage.

### Comparative Example 1

4, 4', 4"-tris(N,N-(2-naphthyl)phenylamino)triphenylamine (2-TNATA) was used as a hole injecting agent, and otherwise in the same manner as Example 2, an organic electroluminescence element was prepared. 2-TNATA has a glass transition temperature of 110°C, and a decomposition temperature of not less than 410°C.

The characteristics of the organic electroluminescence element were examined at a fixed current and at a fixed luminance. The results are shown in Table 1. The voltage-luminance characteristics of the organic electroluminescence element are shown in Fig. 5, and the voltage-current density characteristics of the organic electroluminescence element are shown in Fig. 6. It was found that a higher voltage was needed to drive this organic electroluminescence element than that of Example 2.

**Table 1**

| | Characteristics | | | | |
|---|---|---|---|---|---|
| | At fixed current of 25 mA/cm² | | | | At fixed luminance of 100 cd/m² |
| Organic EL Element | Power Efficiency of Emmission (1m/W) | Current Efficiency of Luminance (cd/A) | Luminance (cd/m²) | Voltage (V) | Voltage (V) |
| Example 2 | 1.74 | 3.15 | 786.33 | 5.69 | 4.19 |
| Comparative 1 | 1.82 | 3.52 | 879.07 | 6.08 | 4.34 |

### Example 3

### (Synthesis of N,N,N'-triphenyl-1,4-phenylenediamine (10))

100.0 g of N,N'-diphenyl-1,4-phenylenediamine, 78.4 g of iodobenzene, 265.5 g of potassium carbonate and 77 g of copper powder and mesitylene as a reaction solvent were placed in a 2 L capacity four necked glass flask, and the reaction was carried out at a temperature of 160°C for 10 hours under a nitrogen atmosphere. After the reaction, the resultant reaction mixture was extracted with toluene and the toluene solution was concentrated. Ethanol was added to the resulting concentrate and the resulting mixture was stirred for a little while, followed by removing resulting precipitates by filtration.

The filtrate was concentrated to provide viscose liquid, to which a mixed solvent of toluene/hexane was added, and the resulting mixture was subjected to silica gel chromatography. The obtained fractions containing the reaction product were concentrated to provide viscose liquid, to which hexane was added. After the mixture was stirred for a little while, the resulting precipitates were collected by filtration, and dried, to provide 37.5 g of the desired N,N,N'-triphenyl-1,4-phenylenediamine as white solid. The yield was 29%.

### (Synthesis of 1,4-bis[N-(4-diphenylaminophenyl)- N-phenylamino]-naphthalene (8))

26.5 g of N,N,N'-triphenyl-1,4-phenylenediamine, 9.0 g of 1,4-dibromonaphthalene, 6.0g of sodium t-butoxide and 0.008 g of palladium t-butylphosphinebromide dimmer were dissolved in 200 mL of o-xylene. The reaction was carried out at a temperature of 70°C for 5 hours under a nitrogen atmosphere. After the reaction, the resulting reaction mixture was extracted with a mixture of water/toluene and the organic layer was dehydrated over anhydrous magnesium sulfide, followed by concentration. The resulting concentrate was fractionated by chromatography on a column of silica gel and the desired fractions were concentrated under reduced pressure to provide solid. The solid was recrystallized from toluene and was then subjected to sublimation purification, thereby providing 3.9 g of 1,4-bis[N-(4-diphenylamino-phenyl)-N-phenylamino]naphthalene (1) as yellow solid. The yield was 16%.

| Elemental analysis (%): | | | |
|---|---|---|---|
| | C | H | N |
| Calculated: | 87.44 | 5.53 | 7.03 |
| Measured: | 87.31 | 5.51 | 7.18 |

| | | | |
|---|---|---|---|
| Molecular weight by mass analysis: 796.5 | | | |

### FT-IR (Fourier transform infrared spectroscopy, KBR method):

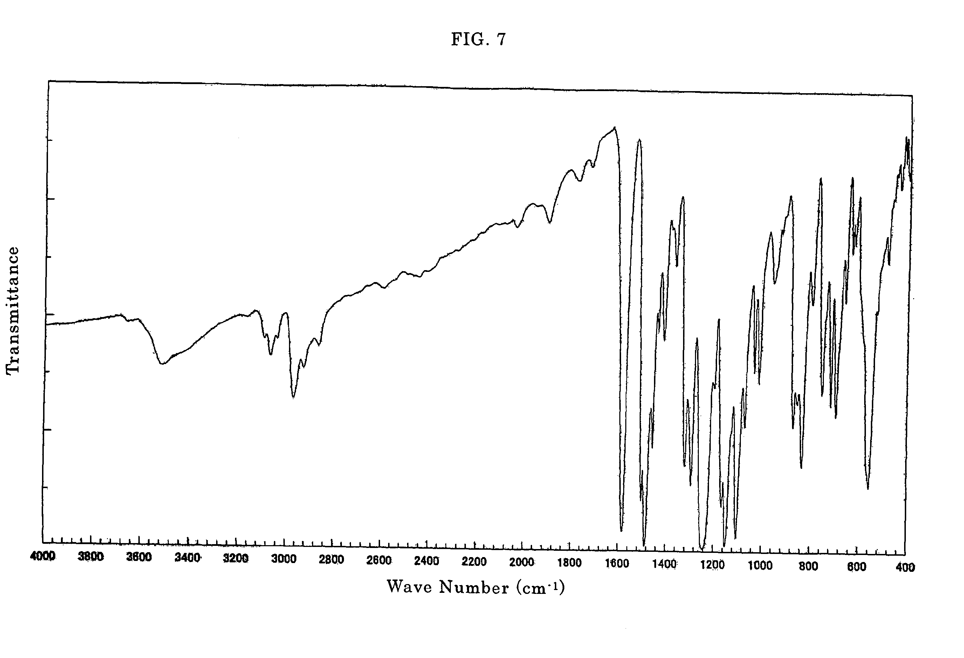

The spectrum is shown in Fig. 7.

### Differential scanning calorimetry (DSC):

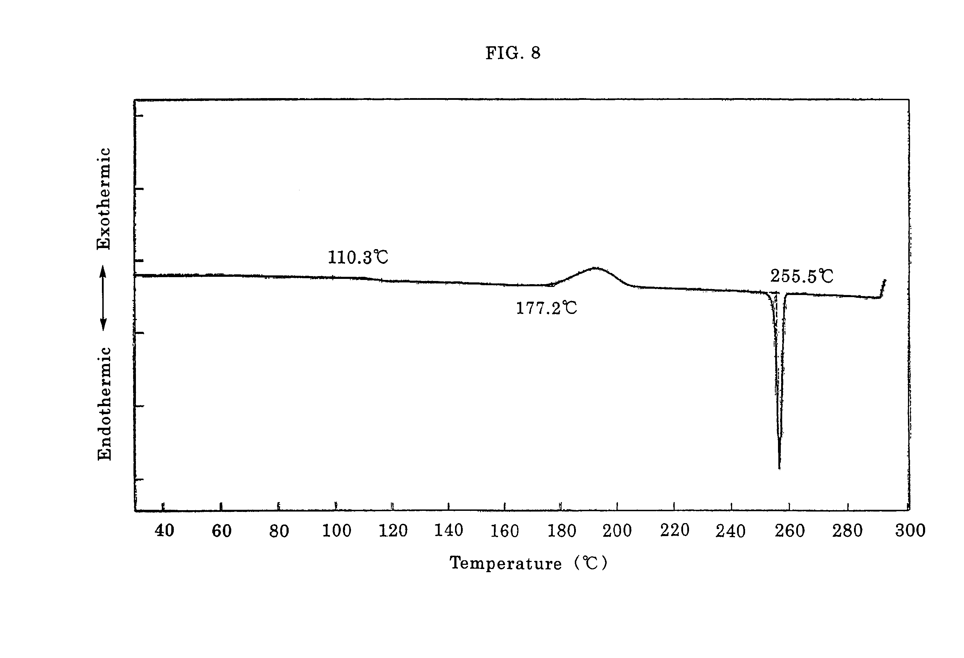

As the DSC chart is shown in Fig. 8, the compound was found to have a glass transition temperature (Tg) of 110.3°C, a crystallization temperature of 177.2°C, and a melting point of 255.5°C, indicating that the compound is capable of forming stable amorphous film at ordinary temperature or higher.

### Thermogravimetric measurement/Differential calorimetry (TG/DTA):

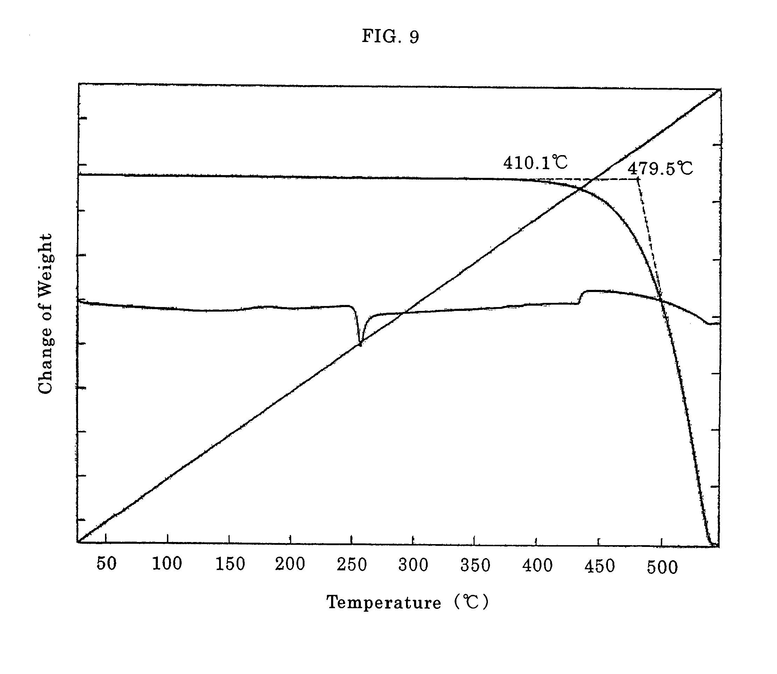

As the results of measurement is shown in Fig. 9, the compound was found to have a decomposition temperature of not less than 400°C, indicating that the compound is excellent in heat stability.

### Cyclic voltammetry (CV):

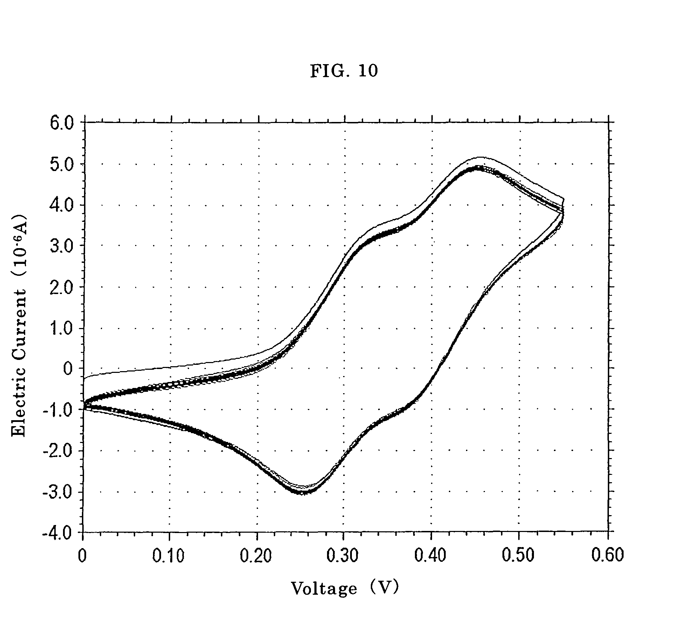

As the CV chart is shown in Fig. 10, the compound was found to have an oxidation potential of 0.29 V (vs. Ag/Ag⁺).

### Example 4

A hole injecting layer (50nm thick) formed of 1,4-bis-[N-(4-diphenylaminophenyl)-N-phenylamino]naphthalene was laminated on an ITO transparent electrode (anode). A hole transporting layer (10 nm thick) was formed of 4,4'-bis(N-(1-naphthyl)-N-phenylamino)]-biphenyl (α-NPD) on the hole injecting layer. An emission layer (75nm thick) was formed of tris(8-quinolinol) aluminum (Alq₃) on the hole transporting layer by a vacuum evaporation method. Further, lithium fluoride (0.5 nm thick) and aluminum (100 nm thick) were vacuum evaporated in this order as a cathode on the emission layer, thereby preparing an organic electroluminescence element.

The voltage-luminance characteristics of the organic electroluminescence element were measured, and it was found that emission started at a voltage of 2.5 V and when the luminance was 1000 cd/m², the driving voltage was 9.0 V.

### Comparative Example 2

An α-NPD layer (60 nm thick) was formed as a hole transporting layer, and otherwise in the same manner as Example 4, an organic electroluminescence element was prepared. The voltage-luminance characteristics were measured, and it was found that emission started at a voltage of 5.0 V, and when the luminance was 1000 cd/m², the driving voltage was 14.0 V. The oxidation potential of α-NPD is 0.48 V (vs. Ag/Ag⁺), and the ionization potential is 5.46 V.

### Example 5

### (Synthesis of 9,10-bis[N-(4-diphenylaminophenyl)-N-phenylamino]-anthracene)

A hexane solution of 40.0 g of N,N,N'-triphenyl-1,4-phenylenediamine, 15.9 g of 9,10-dibromoanthracene, 21.9 g of sodium t-butoxide, 0.10 mg of palladium acetate and 0.38 g of tri-t-butylphosphine were dissolved in 300 mL of o-xylene. The reaction was carried out at a temperature of 70°C for 8 hours under a nitrogen atmosphere. After the reaction, water was added to the resulting reaction mixture and the resulting red precipitates were collected by filtration. The precipitates were dissolved in hot toluene, and the resulting solution was filtered while hot to remove impurities therefrom, followed by cooling, to provide 24.7 g of 9,10-bis[N-(4-diphenylaminophenyl)-N-phenylamino]anthracene as red solid. The yield was 24%.

| Elemental analysis (%): | | | |
|---|---|---|---|
| | C | H | N |
| Calculated: | 87.84 | 5.43 | 6.73 |
| Measured: | 87.75 | 5.40 | 6.85 |

| | | | |
|---|---|---|---|
| Molecular weight by mass analysis: 846.7 | | | |

### Example 6

A hole injecting layer was formed of 9,10-bis[N-(4-diphenyl-aminophenyl)-N-phenylamino]anthracene as a hole injecting agent, and otherwise in the same manner as Example 4, an organic electroluminescence element was prepared. The voltage-luminance characteristics were measured, and it was found that emission started at a voltage of 2.6 V, and when the luminance was 1000 cd/m², the driving voltage was 9.4 V.

### Example 7

### (Synthesis of 1,4-bis[N-(4-diphenylaminophenyl)-N-p-tolylamino]-naphthalene))

N,N'-di-p-tolyl-1,4-phenylenediamine was used in place of N,N'-diphenyl-1,4-phenylenediamine, and otherwise in the same manner as Example 3, N-phenyl-N',N'-di-p-tolyl-1,4-phenylenediamine was obtained, which was then reacted with 1,4-dibromonaphthalene in the same manner as Example 3, to prepare 1,4-bis[N-(4-diphenylamino-phenyl)-N-p-tolylamino] naphthalene.

| Elemental analysis (%): | | | |
|---|---|---|---|
| | C | H | N |
| Calculated: | 87.30 | 5.87 | 6.83 |
| Measured: | 87.11 | 5.76 | 7.13 |

| | | | |
|---|---|---|---|
| Molecular weight by mass analysis: 824.7 | | | |

### Example 8

A hole injecting layer was formed of 1,4-bis[N-(4-diphenyl-aminophenyl)-N-p-tolylamino]naphthalene as a hole injecting agent, and otherwise in the same manner as Example 4, an organic electroluminescence element was prepared. The voltage-luminance characteristics were measured, and it was found that emission started at a voltage of 2.4 V, and when the luminance was 1000 cd/m², the driving voltage was 9.3 V.

## Claims

1. An aromatic tertiary amine represented by the general formula (I) 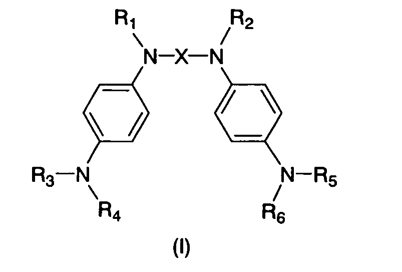 wherein X is an aromatic divalent group represented by the general formula (II), (III) or (IV) 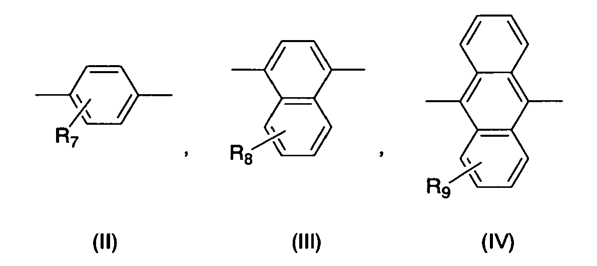 wherein R₇ to R₉ each independently represent a hydrogen atom, an alkyl group of 1 to 6 carbons or an alkoxyl group of 1-4 carbons, and R₁ to R₆ each independently represent a monovalent group (V), (VI), (VII), (VIII), (IX) or (X) 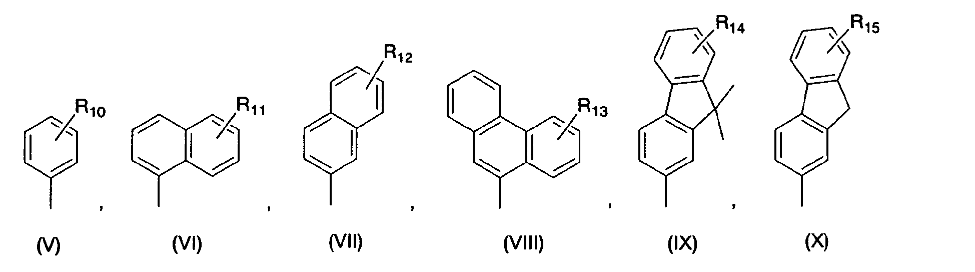 wherein R₁₀ to R₁₅ each independently represent a hydrogen atom, an alkyl group of 1 to 6 carbons or an alkoxyl group of 1 to 4 carbon atoms, provided that all of R₁ to R₆ are each a pheny groups, X is not a 1,4-phenylene group.

2. The aromatic tertiary amine represented by the general formula (I) as claimed in claim 1, wherein R₁, R₂, R₃ and R₅ each independently represent a 1- or 2-naphthyl group.

3. The aromatic tertiary amine represented by the general formula (I) as claimed in claim 1, wherein R₁, R₂, R₃ and R₅ each independently represent a 1- or 2-naphthyl group; R₄ and R₆ each represent a phenyl, a tolyl or a naphthyl group; and the group X is a 1,4-phenylene, a 1,4-naphthalenediyl or a 9, 10-anthracenediyl group.

4. The aromatic tertiary amine as claimed in claim 1, which is represented by the formula

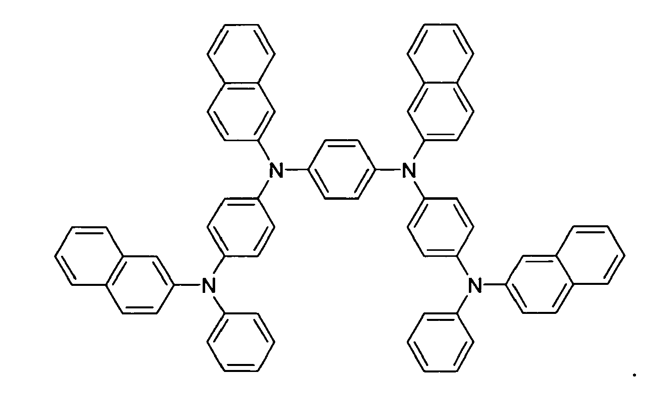

5. The aromatic tertiary amine represented by the general formula (I) as claimed in claim 1, wherein R₁, R₂, R₃, R₄, R₅ and R₆ each represent a phenyl or a tolyl group, provided that all of R₁ to R₆ are each a phenyl group, X is not a 1, 4-phenylene group.

6. The aromatic tertiary amine represented by the general formula (I) as claimed in claim 1, wherein R₁, R₂, R₃, R₄, R₅ and R₆ each represent a phenyl or a tolyl group, and the group X is a 1,4-phenylene, a 1,4-naphthalenediyl or a 9,10-anthracenediyl group, provided that all of R₁ to R₆ are each a phenyl group, X is not a 1,4-phenylene group.

7. The aromatic tertiary amine as claimed in claim 1, which is represented by the formula

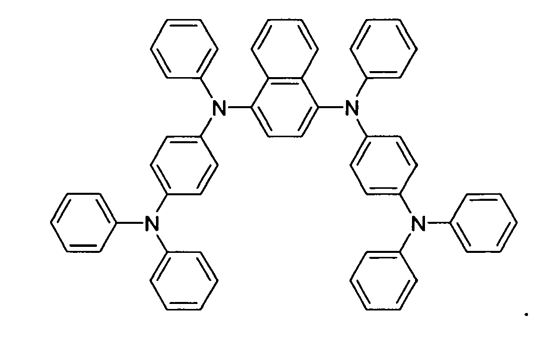

8. An organic electronic functional material which comprises an aromatic tertiary amine as claimed in any one of claims 1 to 7.

9. A hole injecting agent which comprises an organic electronic functional material as claimed in claim 8.

10. An organic electroluminescence element having a hole injecting layer comprising a hole injecting agent as claimed in claim 9.
